# EUROPÄISCHE PATENTANMELDUNG

(11) **EP 2 873 390 A1**
(43) Veröffentlichungstag der Anmeldung: **20.05.2015**
(21) Anmeldenummer: 14191027.3
(22) Anmeldetag: 30.10.2014
(51) Int. Cl.: A61F 2/00, B21C 23/06, B21C 23/08, B21C 23/10, B21C 25/02, B21C 25/04, B21C 25/08, C22C 23/00, C22F 1/06

(54) **Halbzeug und hochfestes degradierbares Implantat daraus**

(30) Priorität: 18.11.2013 US 201361905299 P
(71) Anmelder: Biotronik AG, 8180 Bülach (CH)
(72) Erfinder: Bayer, Ullrich, 18209 Bad Doberan (DE); Anopuo, Okechukwu, 18119 Rostock (DE); Block, Bernd, 18211 Ostseebad Nienhagen (DE)
(74) Vertreter: Lindner-Vogt, Karin L.

(57) **Zusammenfassung**

Die Erfindung betrifft ein Halbzeug für ein Implantat, umfassend oder bestehend aus einem Bereich einer Magnesiumlegierung, der durch einen Korngrößengradienten der Magnesiumlegierung zwischen zwei gegenüberliegenden Oberflächen von ≤ 3 µm bis ≥ 8 µm bezogen jeweils auf die mittlere Korngröße gekennzeichnet ist. Die Erfindung betrifft auch Implantate, hergestellt aus diesem Halbzeug. Außerdem betrifft die Erfindung die Verwendung des erfindungsgemäßen Halbzeuges zur Herstellung von entsprechenden Implantaten sowie ein Verfahren zur Herstellung erfindungsgemäßer Halbzeuge.

## Beschreibung

Die Erfindung betrifft ein Halbzeug für ein Implantat, umfassend oder bestehend aus einem Bereich einer Magnesiumlegierung, der durch einen Korngrößengradienten der Magnesiumlegierung zwischen zwei gegenüberliegenden Oberflächen von ≤ 3 µm bis ≥ 8 µm bezogen jeweils auf die mittlere Korngröße gekennzeichnet ist. Die Erfindung betrifft auch Implantate, hergestellt aus diesem Halbzeug. Außerdem betrifft die Erfindung die Verwendung des erfindungsgemäßen Halbzeuges zur Herstellung von entsprechenden Implantaten sowie ein Verfahren zur Herstellung erfindungsgemäßer Halbzeuge.

Insbesondere für orthopädische Implantate besteht ein ständiger Bedarf nach Implantatmaterialien, die hohen Anforderungen an mechanische Belastung, wie z. B. Zug-, Biege- und Druckfestigkeit entsprechen und geeignet sind für Einsätze mit hohen Flächenpressungen. Beispiele für solche orthopädische Implantate sind kanülierte Knochenschrauben, Kirschnerdrähte sowie Implantate für die Wirbelsäulenchirurgie wie vertebral body stenting (VBS).

Aus einer Vielzahl von Gesichtspunkten ist es wünschenswert, dass die Implantate aus Magnesiumlegierungen bestehen können, da diese Legierungen in einer Vielzahl von Einsatzgebieten besonders gute Verarbeitbarkeit und/oder Verträglichkeitseigenschaften zeigen. Insbesondere ist es bevorzugt, wenn die Legierung bioresorbierbar ist, so dass sich eine Explantation des Implantates nach dessen Funktionserfüllung erübrigt.

In vielen Bereichen, insbesondere in solchen mit hohen mechanischen Anforderungen an das Implantat, war es bislang nicht möglich, Magnesiumlegierungen einzusetzen. Im Stand der Technik wurden stattdessen z. B. hochfeste Eisenbasislegierungen eingesetzt, die mit Mn, Pd und/oder Pt zur Erhöhung der Degradationsrate legiert sind. Neben langen Degradationszeiten besitzen diese Legierungen den weiteren Nachteil, dass stress shielding-Effekte aufgrund der diesen Legierungen eigenen hohen E-Moduli auftreten und so der eigentlichen Funktion des Implantates - der biologischen Verankerung zum Knochengewebe - nachhaltig entgegen gewirkt wird.

Ein alternatives Material im Stand der Technik sind metallische Gläser auf Basis von Mg, Zn, Ca, die unter anderem den Nachteil von zu geringerer Verformbarkeit und zu geringerer Variationsbreite herstellbaren Halbzeugen aufweisen.

Insbesondere aus dem Gesichtspunkt der Bioabbaubarkeit werden im Stand der Technik auch (nicht-extrudierte) mittelfeste degradierbare Magnesiumlegierungen, wie z. B. WE 43 eingesetzt, die aber entweder nicht die ausreichende Festigkeit für das Einbringen von z. B. Gewinden besitzen oder aber eine zu hohe Degradationsgeschwindigkeit aufgrund von hoher Korngröße aufweisen.

Als weitere alternative Materialkompositionen sind im Stand der Technik Metall-Zementkomposite wie z. B. Eisen/Brushit-Schäume bekannt, die jedoch u.a. den Nachteil einer Partikelgenerierung während der Implantation aufweisen, was die Gefahr nachfolgender lokaler Entzündung erhöht.

Aufgabe der vorliegenden Erfindung war es daher ein auf Basis von Magnesiumlegierungen verbessertes Halbzeug für die Herstellung von Implantaten bereitzustellen. Insbesondere war es interessant, Legierungen einzusetzen, die auch erhöhten mechanischen Anforderungen entsprechen und bevorzugt auch biodegradierbar (bioresorbierbar) sind. Insbesondere war es ein bevorzugtes Ziel, das Einsatzfeld von degradierbaren Implantaten in Bereiche zu erweitern, die bisher nur hochfesten, nicht-degradierbaren Materialien, wie Titan, L605 und 316L zugängig waren.

Die der Erfindung zugrundeliegende Aufgabe wird gelöst durch ein Halbzeug für ein Implantat, umfassend oder bestehend aus einem Bereich aus einer Mg-Legierung, der durch einen Korngrößengradienten der Mg-Legierung zwischen zwei gegenüberliegen Oberflächen von ≤ 3µm bis ≥ 8µm bevorzugt von ≤ 2µm bis ≥ 10µm bezogen jeweils auf die mittlere Korngröße gekennzeichnet ist.

Eine Magnesiumlegierung im Sinne dieses Textes ist eine Legierung, die mindestens einen Anteil von ≥ 80 Gew.% Magnesium umfasst.

Weiter bevorzugte Bestandteile von erfindungsgemäß einzusetzenden Magnesiumlegierungen sind Zn, Ca, Li, Mn und Seltene Erden, bevorzugt zu folgenden Anteilen (jeweils in Gew.% bezogen auf die Gesamtlegierung):

| | |
|---|---|
| Li: | 0,01 - 3,0 |
| Ca: | 0,01 - 2,0 |
| Zn: | 0,01-5,0 |
| Mn: | 0,01-0,5 |
| Nd: | 0,01-1,5 |
| Dy: | 0,01-1,0 |
| Gd: | 0,01-1,0 und/oder |
| Eu: | 0,01-0,5 |

Bevorzugte Magnesiumlegierungen für das erfindungsgemäße Halbzeug sind Beispiele für besonders geeignete Legierungen sind:
1.) WE 43 (3,7 bis 4,3 Gew.% Y; 2,4-4,4 Gew.% Seltene Erden; 0,4-0,7 Gew.% Zr; Rest Magnesium)
2.) AZ 31 (2,5-3,5 Gew.% Al; 0,7-1,3 Gew.% Zn; 0,2-0,4 Gew.% Mn; jeweils max. 0,05 Gew.% Si und Cu; max. 0,04 Gew.% Ca; jeweils max. 0,005 Gew.% Fe und Ni; Rest Magnesium)
3.) AM 60 (5,0-6,0 Gew.% Al; 1,5 -2,5 Gew.% Ca; 0,2-0,5 Gew.% Mn; Rest Magnesium)

Die Korngrößenkennzahl G wird aus der mittleren Anzahl der Körner m bestimmt, welche auf einem Quadratmillimeter Querschnittsfläche der Probe gezählt werden. Die Korngrößenbestimmung wird mit Bildreihentafeln gemäß der ASTM E 112-12 durchgeführt.

Die Ermittlung der Korngröße erfolgt dabei lichtmikroskopisch mit Hilfe von genormten Bildtafeln. Jede dieser schablonenartigen Bildtafeln stellt eine mittlere Korngröße dar. Diese Bildtafeln werden mit der Kornstruktur des geätzten metallographischen Schliffs bei einer 100-fachen Vergrößerung verglichen. Bei der größtmöglichen Übereinstimmung des Schliffes mit einer Bildtafel wird die Korngröße von der Bildtafel abgelesen.

Aufgrund des erfindungsgemäß vorgesehenen Gradienten der Korngröße der erfindungsgemäß einzusetzenden Magnesiumlegierung umfasst das Halbzeug Bereiche unterschiedlicher mechanischer Eigenschaften, die wesentlich durch die Korngröße in der Legierung bestimmt werden. Dabei stellte sich überraschenderweise heraus, dass die erfindungsgemäßen Halbzeuge und daraus hergestellte Implantate eine gute Eigenschaftskombination aus Streckgrenzen- und Zugfestigkeitswerten bei ausreichender Bruchdehnung aufweisen. Dabei bieten sich die Bereiche mit der größeren mittleren Korngröße für den Einsatz in Bereichen an, in denen die (späteren) Implantate besonders mechanisch und/oder korrosiv beansprucht sind.

Dies ermöglicht es, die erfindungsgemäßen Halbzeuge für Implantate für die Anwendung in der Orthopädie einzusetzen, bei denen hohe Anforderungen an die Zug-, Biege- und Druckfestigkeit sowie hohe Flächenpressungen bestehen.

Beispiele dafür sind kanülierte Knochenschrauben, Kirschnerdrähte sowie Implantate für die Wirbelsäulenchirurgie wie vertebral body stenting (VBS). Die aus dem erfindungsgemäßen Halbzeugmaterial entwickelten Implantate können sowohl in der Wirbelkörperchirurgie als auch in der Knochenfixierung und -Stabilisierung mit oder ohne von die Osteosynthese beschleunigenden Substanzen zum Einsatz kommen.

Grundsätzlich können die erfindungsgemäßen Halbzeuge oder die daraus hergestellten Implantate auch mit Substanzen bestückt werden, die die Funktion des Implantates unterstützen.

Dies können zum Beispiel calziumphosphathaltige Verbindungen wie Hydroxylapatit (HAP) oder bone morphogenetic proteins (bmp's) sein mit denen derartige Implantate hinsichtlich einer beschleunigten Osteosynthese oberflächenfunktionalisiert werden.

Dies können auch oberflächenaktive Phosphonate sein. So beeinflusen Copolymere wie 4-Vinylpyridin mit Vinylbenzylphosphonat oder Dimethyl (2-methacryloyloxy-ethyl) phosphonat die Osseointegration positiv und behindern auch noch die Adhäsion von Bakterien an den Implantatoberflächen. Legierungstechnisch bieten sich vor allem die Elemente Ca und Zn an, die bei fortschreitender Degradation des resorbieren Magnesiumimplantates die Integration in die Knochensubstanz befördern.

Erfindungsgemäß bevorzugt ist, dass das erfindungsgemäße Halbzeug ein Flächengebilde oder ein Hohlprofil darstellt.

Ein Flächengebilde im Sinne dieses Textes ist ein Körper, der über zwei annähernd gleich große Hauptflächen (Vorder- und Rückseite) verfügt, wobei diese beiden Hauptflächen einander gegenüberstehen und der Körper sich im Wesentlichen in nur zwei Raumrichtungen erstreckt.

Ein Hohlprofil im Sinne dieses Textes ist ein Körper, der einen abgeschlossenen Hohlraum (z. B. Hohlkugel) oder einen offenen Hohlraum (z. B. Röhre) umfasst.

Bevorzugte Hohlprofile im Sinne dieses Textes sind dabei offene Zylinder.

Bei den bevorzugten erfindungsgemäßen Halbzeugen, die ein Flächengebilde darstellen, ist erfindungsgemäß vorgesehen, dass der Korngrößengradient von der einen zur anderen Hauptfläche des Flächengebildes läuft. Im Falle von Hohlprofilen ist es erfindungsgemäß vorgesehen, dass der Korngrößengradient von der Innenfläche zur Außenfläche des Körpers läuft.

Hierdurch entstehen Flächen, die unterschiedliche mechanische Eigenschaften aufweisen, die aber für das Halbzeug und das daraus gefertigte Implantat in positiver Weise zusammenwirken. Außerdem ist es möglich, Eigenschaften einer dieser Flächen für bestimmte Zwecke auszunutzen (vgl. weiter unten).

Erfindungsgemäß bevorzugt ist es, dass die Magnesiumlegierung bioresorbierbar ist.

Bioresorbierbar bedeutet im Sinne der vorliegenden Erfindung, dass beim Einbau in den menschlichen oder tierischen Körper, bevorzugt in den menschlichen Körper die Legierung mit einer Geschwindigkeit von 1 µm bis 3 µm/Woche abgebaut wird. Das bedeutet, dass ein Implantat mit einer Wandstärke von 150 µm bei einer Abbaugeschwindigkeit von 1 µm/Woche in 75 Wochen vollständig abgebaut wird. Bei einer Abbaurate von 3 µm/Woche wäre dieses Implantat nach bereits 25 Wochen vollständig abgebaut.

Überraschenderweise lassen sich mit den erfindungsgemäßen bioresorbierbaren Halbzeugen Festigkeitseigenschaften von nicht-degradierbaren Legierungen, wie z. B. Titan und Edelstahl 316L im ausreichenden Maße erreichen. Dies ist aufgrund der durch das Hauptlegierungselement Mg vorgegebenen physikalischen Gegebenheiten, wie Gitterstruktur und atomare Bindungskräfte nicht selbstverständlich zu erwarten gewesen.
Mit dem nun erfindungsgemäß bereitgestellten bioresorbierbaren Halbzeugen lassen sich Implantate herstellen, die aufgrund ihrer mechanischen Eigenschaften so ausgestattet sind, dass Kliniker, die mit der Implantation von nicht-degradierbaren orthopädischen Implantaten vertraut sind, keine wesentlichen Änderungen in der Operationsmethodik vornehmen müssen. Dies beinhaltet auch die Verwendbarkeit der üblicherweise vorhandenen klinischen Operationsbestecke wie Schraubendreher, Drehmomentschlüssel und Zangen.

Bevorzugt ist dabei ein erfindungsgemäßes Halbzeug, das in einem Extrusionsverfahren hergestellt ist.

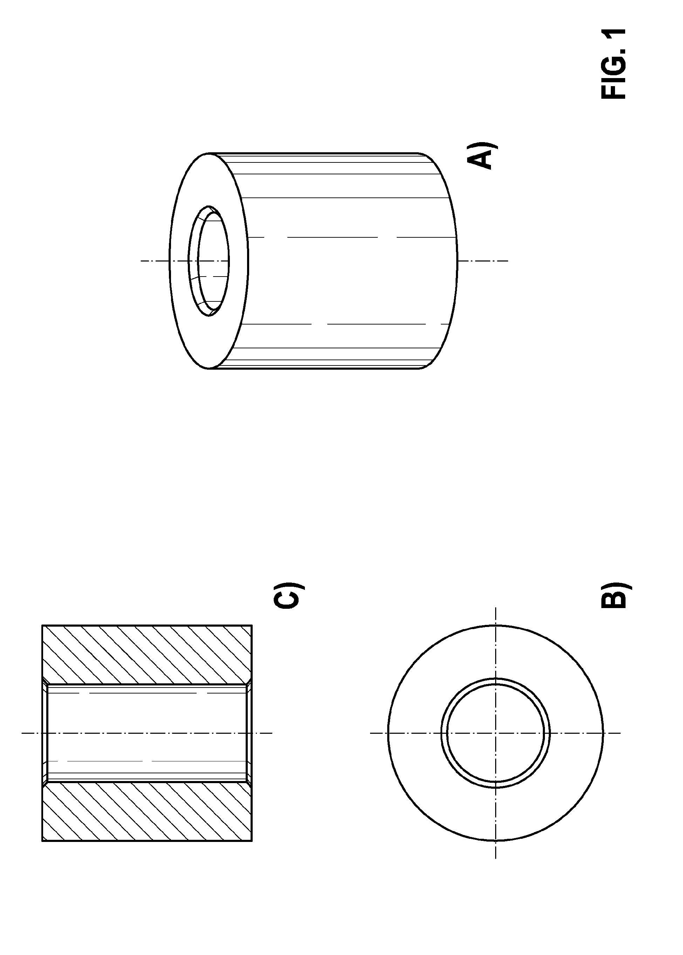

Über ein entsprechend ausgestaltetes Extrusionsverfahren lassen sich dabei besonders günstig und effektiv die erfindungsgemäßen Halbzeuge herstellen. Geeignete bevorzugte Maßnahmen sind in diesem Zusammenhang:
- Eine spezielle Form des umzuformenden Rohlings in Form eines Hohlzylinders, eine bevorzugte Variante ist in Fig. 1a bis c abgebildet.
- Eine geeignete Werkzeuggeometrie für das Extrusionswerkzeug, mit der spezifische Umformgrade über den Querschnitt des Halbzeuges erzielt werden, die wiederum Gradienten in der Korngröße zwischen Innen- und Außenseite generieren. Hier sind insbesondere folgende Maßnahmen zu nennen.

Im Besonderen geht es darum, unterschiedlich geformte Kanten im Inneren des Werkzeuges zu realisieren. Bei scharfen Kanten, d.h. Kanten mit kleinem Krümmungsradius (z. B. R= 0,01 mm) wird der Rohling mehr umgeformt als bei einer Kante mit großem Krümmungsradius (z. B. R= 5,0 mm). D.h., dass beim Fließen des Magnesiums (bzw. der Magnesium-Legierung) um eher "eckenförmige" Strukturen die ursprünglich vorhandene Gefügestruktur in größerem Maße beeinflusst (zertrümmert) wird als bei der Verformung um mehr runde Werkzeugstrukturen. An diesen Stellen werden die Körner weniger verdichtet, die ursprünglich vorhandene Versetzungsdichte wird im Vergleich zur Verformung um scharfe Kanten weniger erhöht und die ursprünglich vorhandene mittlere Korngröße bleibt relativ unverändert.
- Eine spezifische Umformgeschwindigkeit zwischen 0,01 s⁻¹ und 35 s⁻¹.
- den Verzicht auf eine nachgeschaltete Wärmebehandlung oder eine spezielle Wärmebehandlung nach dem Extrusionsprozess zur Stabilisierung des Mikrostrukturgradienten über den Querschnitt des extrudierten Produktes. Bevorzugte Parameter für diese Wärmebehandlung sind 200°C bis 500°C für eine Zeitdauer von 3 bis 180 Minuten.

Dadurch kann eine Gefügeausbildung erzielt werden, die sich durch extrem feine Korngrößen (mittlerer Korndurchmesser ≤ 3 µm (bevorzugt ≤ 2 µm) auf der einen Seite des Halbzeugquerschnitts und Korngrößen ≥ 8 µm (bevorzugt ≥ 10 µm, besonders bevorzugt ≥ 15 µm) auf der gegenüberliegenden Halbzeugseite auszeichnen.

Dabei sind die beiden Halbzeugseiten (oben und unten bzw. innen und außen) durch die jeweilige Anordnung der Umformwerkzeuge austauschbar.

Dementsprechend ist ein erfindungsgemäßes Halbzeug bevorzugt, wobei das Halbzeug ein Hohlprofil in Form eines Zylinders ist und die Außenfläche des Zylindermantels die Legierung mit der größeren mittleren Korngröße umfasst oder daraus besteht und die Innenfläche des Zylindermantels die Legierung mit der kleineren mittleren Korngröße umfasst oder daraus besteht.

Ebenso kann für andere Zwecke ein erfindungsgemäßes Halbzeug bevorzugt sein kann, wobei das Halbzeug ein Hohlprofil in Form eines Zylinders ist und die Außenfläche des Zylindermantels die Legierung mit der kleineren mittleren Korngröße umfasst oder daraus besteht und die Innenfläche des Zylindermantels die Legierung mit der größeren mittleren Korngröße umfasst oder daraus besteht.

Der Extrusionsprozess wird bevorzugt mit einem vorher lösungsgeglühten Material durchgeführt. In diesem Zustand weist das Gefüge große Körner und keine Ausscheidungen auf. Das bedeutet, dass alle Legierungselemente in der Matrix gelöst sind. Der anschließende Extrusionsprozess wird bei Temperaturen und Umformdrücken geführt, die einerseits eine erneute Ausscheidungsbildung unterdrücken und andererseits zu submikroskopischen Korngrößen führt. Von besonderer Bedeutung ist dabei:
- dass die Temperatur des Beginns der dynamischen Rekristallisation unterschritten wird,
- dass durch eine spezielle Werkzeuggeometrie Bereiche unterschiedlich große Verformungsgrade über den Bauteilquerschnitt entstehen und/oder
- dass die nachfolgende Rekristalliationsglühung hinsichtlich Zeit und Temperatur so geführt wird, dass die Zonen mit unterschiedlichen Verformungsgraden so rekristallisieren, dass der jeweils verschiedene Umformgrad erhalten bleibt.

Eine unterschiedliche Verteilung der Korngrößen über den Bauteilquerschnitt ist die Folge sowie eine homogene Ausbildungsverteilung.

Unterschiedliche Magnesiumlegierungen lassen sich mit dem geeigneten Extrusionswerkzeug auf die gewünschten Korngrößengradienten einstellen.

Der Korngrößengradient führt dazu, dass - wie beschrieben - überraschend gute mechanische Eigenschaften für die erfindungsgemäßen Halbzeuge erreicht werden können.

Dementsprechend ist erfindungsgemäß bevorzugt ein Halbzeug, wobei dieses im Bereich der Magnesiumlegierung eine Zugfestigkeit von ≥ 400 bevorzugt ≥ 440 MPa und/oder eine Streckgrenze von ≥ 225 bevorzugt ≥ 250 MPa besitzt und/oder eine Bruchdehnung von ≥ 4%, bevorzugt ≥ 4,5% besitzt.

Diese bevorzugten mechanischen Eigenschaften machen die erfindungsgemäßen Halbzeuge bzw. die daraus hergestellten Implantate auch in ihrer bevorzugten bioresorbierbaren Form für eine Vielzahl von Anwendungen zugänglich, die bislang insbesondere für bioresorbierbare Magnesiumlegierungen verschlossen waren.

Bevorzugt ist in dem Zusammenhang, dass die erfindungsgemäßen Halbzeuge bezogen auf das daraus herzustellende Implantat endkonturnah hergestellt werden, so dass nur wenig Fertigungsaufwand zur Herstellung des eigentlichen Implantates nötig ist.

Erfindungsgemäß bevorzugt ist ein Halbzeug, wobei die Streckgrenze des Bereiches der Magnesiumlegierung mit der kleineren mittleren Korngröße um ≥ 40 MPa, bevorzugt ≥ 45 MPa größer ist als die Streckgrenze des Bereiches der Mg-Legierung mit der größeren mittleren Korngröße.

Durch diese verhältnismäßig großen Unterschiede in der Streckgrenze lassen sich im Bereich der Magnesiumlegierung mit der größeren mittleren Korngröße Ausgestaltungen verwirklichen, die eine nachträgliche Warmverformung dieses Bereiches erforderlich machen oder wenigstens durch eine solche begünstigt werden. So ist beispielsweise das Vorsehen eines Gewindes, insbesondere eines Außengewindes nur durch die erfindungsgemäße Ausgestaltung des erfindungsgemäßen Halbzeuges möglich, ohne dass die mechanischen Eigenschaften zu stark verschlechtert werden.

So ist es beispielsweise möglich, Schrauben herzustellen, die ein um 30% erhöhtes Drehmoment beim Einschrauben in den Knochen ermöglichen, als Knochenschrauben, deren Gewinde spanend und ohne die spezielle Ausgestaltung des erfindungsgemäßen Halbzeuges (Korngrößengradient) aus dem gleichen Legierungsmaterial hergestellt wurden.

Teil der Erfindung ist auch ein Implantat hergestellt oder herstellbar aus einem erfindungsgemäßen Halbzeug. Dieses Implantat besitzt die oben beschriebenen Vorteile, insbesondere in seiner bevorzugten Form der Bioresorbierbarkeit, so dass es nach Erfüllung seiner Aufgaben nicht mehr explantiert werden muss.

Bevorzugt ist ein erfindungsgemäßes Implantat, das im Bereich der Magnesiumlegierung mit der größeren mittleren Korngröße ein Gewinde oder eine andere die reale Oberfläche vergrößernde Topographie aufweist. Dies ist vor allem dort von Vorteil, wo eine große Oberfläche für einen intensiveren Flächenkontakt zum umgebenden Knochen gewünscht wird. Auf diese Weise erhöht sich die Inkorporationsrate des Implantates in den Knochen und dieser kann bereits zu einem früheren Zeitpunkt mechanisch belastet werden.

Bevorzugt ist ein erfindungsgemäßes Implantat ausgewählt aus der Gruppe bestehend aus innenkanülierten Knochenschrauben, pico-Pin Schrauben, Knochenklammern, Marknägeln, Intramedschienen, Cerclagedraht, die Wirbelsäule versteifenden Drahtkäfigen. Teil der Erfindung ist auch die Verwendung eines erfindungsgemäßen Halbzeuges zur Herstellung eines Implantates.

Ferner ist Teil der Erfindung ein Verfahren zur Herstellung eines erfindungsgemäßen Halbzeuges, umfassend die Schritte
a) Bereitstellen einer Mg-Legierung,
b) Bereitstellen einer Extrusionsvorrichtung für die Legierung, die so ausgestaltet ist, dass beim Extrudieren zwei gegenüberliegende Flächen aus Legierungsbestandteilen entstehen, die einer unterschiedlichen Extrusionsgeschwindigkeit (und damit einen unterschiedlichen Verformungsgrad) unterworfen waren und
c) Extrudieren der Legierung mittels der Vorrichtung.

Durch eine spezielle für die Erfindung charakteristische Anordnung von Innen- und Außenwerkzeugen (bzw. im Bereich für die Ober- und Unterfläche des extrudierten Gegenstandes) werden die unterschiedlichen Korngrößen über den Bauteilquerschnitt hergestellt.

Bevorzugte erfindungsgemäßen Verfahren hergestellte Halbzeuge sind dabei rotationssymetrische Hohlprofile.

Teil der Erfindung ist auch die Herstellung eines erfindungsgemäßen Implantates aus einem erfindungsgemäßen Halbzeug.

Generell lässt sich anmerken, dass die in medizinischen Anwendungen auftretenden Belastungsfälle, insbesondere für die bevorzugten Implantate mechanische Eigenschaften erfordern, die man vorher nur durch Einsatz permanenter, d.h. nicht-degradierbarer (nichtbioresorbierbarer) Metalle, wie CoCr, Edelstahl 316L und Titan erreichen konnte. Insbesondere die Flächenpressungen, die im Gewinde von fest angezogenen Knochenschrauben anliegen sowie die Biegesteifigkeit von Knochenklammern machen den Einsatz von höherfesten Materialien erforderlich. Das erfindungsgemäße bevorzugte Implantat (bioresorbierbar) kann während und nach dem Prozess der Knochenheilung seinen Degradationsprozess (Bioresorption) ohne negative Folgen für den Körper fortsetzen. Eine Reoperation zur Entfernung des Implantates ist nicht erforderlich.

Ferner ist ein allgemeiner Vorteil der erfindungsgemäß einzusetzenden Implantate, dass Magnesiumlegierungen nur über einen verhältnismäßig geringen E-Modul, von z. B. 44 GPa verfügen. Dieser verhindert stress shielding-Effekte, die bei konventionellen hochmoduligen Implantatsmaterialien zur negativen Wechselwirkung mit der Knochenoberfläche führen. Ferner ermöglichen die erfindungsgemäßen Halbzeuge und die daraus hergestellten Implantate den Einsatz in Verwendungen, bei denen es auf die Resorbierbarkeit des Implantates ankam, die bislang nur durch degradierbare Polymere abgedeckt werden konnten.

Darüber hinaus bieten Hohldrähte und Hohlstangen als erfindungsgemäße Implante die Möglichkeit der Innenbefüllung mit z. B. die osteosynthesebeschleunigenden Substanzen, bevorzugt z. B. Hydroxylapatit und/oder BMPs (bone morphogenetic proteins).

### Beispiele

### Eingesetztes Werkzeug und spezielle Hinweise:

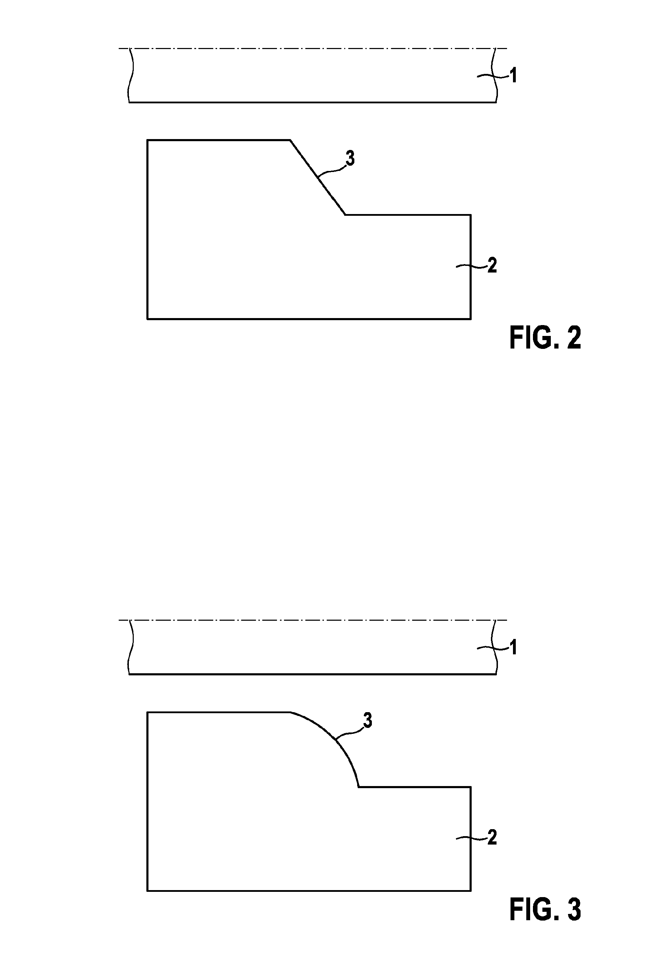

Die Fig. 2 und Fig. 3 zeigen zwei schematische Halbschnitte von Außenteilen des eingesetzten Umformwerkzeuges. In Fig. 2 wird der scharfe Krümmungsradius von 0,01 mm in Fig. 4 der große Krümmungsradius von 5,0 mm dargestellt. Bei Verwendung eines Werkzeuges gemäß Fig. 3 für die Extrusion erfährt das unmittelbar an der scharfen Kante (3) der Matrize (2) vorbeifließende Magnesium eine höhere Verformung als das Materialvolumen das sich weiter zum Innendorn (1) hin befindet. Daraus entstehen die oben genannten Effekte hinsichtlich eines Korngrößengradienten. Die mittlere Korngröße nimmt bei diesem Beispiel also von der Außenseite zur Innenseite des extrudierten Rohres zu.

Unter Verwendung des in Fig. 3 abgebildeten Werkzeuges geschieht der gegenteilige Effekt. Am großen Krümmungsradius (3) der Matrize (2) ist das Magnesium in der Lage ohne wesentliche Verformung vorbei zu fließen. Die ursprünglich vorhandene Korngröße bleibt in dieser der Rohraußenseite nahen Region nahezu vollständig erhalten. Die weiter innen zur Rohrmitte befindlichen Volumina werden durch die Presswirkung des Innendoms (1) stärker verformt. Daraus resultiert ein Gradient in der mittleren Korngröße von innen nach außen. Das führt zu den in diesem Text beschriebenen relativ großen Körnern im Bereich der Rohraußenwand. Diese lassen wiederum eine anschließende Weiterverformung der Rohraußenoberfläche (z. B. mittels Gewindewalzen) zu. Diese Variante kommt also bei der Herstellung von innenkanülierten Knochenschrauben zur Anwendung.

Die erstgenannte Variante -mit den kleineren Körnern außen- kommt bei Produkten zur Anwendung die eine härte Außenoberfläche erforderlich machen. Dies sind z. B. Knochennägel, Drahtkäfige für die Wirbelsäule oder Cerglagedraht deren Hauptbelastung bei der Implantation auf den Außenoberflächen liegt.

### Beispiel 1:

### Ausgangsmaterial: Mg-Legierung WE 43

Vorwärts-Hohlfließpressen einer Hülse bei einer Presstemperatur von 350°C, einer Pressgeschwindigkeit von 5 mm/min ohne nachfolgende Wärmebehandlung
Das entstehende Rohr hat folgende Abmessungen:

| | |
|---|---|
| Länge (L) | = 60 mm |
| Außendurchmesser (AD) | = 3,1 mm |
| Innendurchmesser (ID) | = 1,5 mm |
| Wandstärke (WD) | = 0,8 mm |

Aus dem so prozessierten Rohr werden mittels nachfolgender spanender oder spanloser Bearbeitung der Außenoberfläche innenkanülierte Kortikalisschrauben und/oder innenkanülierte Spongiosaschrauben hergestellt. Der Gewindeaußendurchmesser beträgt dabei 3,0 mm, der Durchmesser der Innenbohrung beträgt 1,5 mm. Dadurch wird eine Fixation von Fragmenten eines Knochenbruchs und eine nachfolgende Knochenheilung ohne Reintervention möglich.

### Beispiel 2:

### Ausgangsmaterial: Mg-Legierung WE 43

Vorwärts-Hohlfließpressen einer Hülse bei einer Presstemperatur von 350°C, einer Pressgeschwindigkeit von 5 mm/min ohne nachfolgende Wärmebehandlung
Das entstehende Rohr hat folgende Abmessungen:

| | |
|---|---|
| Länge (L) | = 60 mm |
| Außendurchmesser (AD) | = 1,8 mm |
| Innendurchmesser (ID) | = 0,8 mm |
| Wandstärke (WD) | = 0,5 mm |

Aus dem so prozessierten Rohr werden mittels nachfolgender spanender oder spanloser Bearbeitung der Außenoberfläche innenkanülierte Kortikalisschrauben und/oder innenkanülierte Spongiosaschrauben für Kleinfragmente im Fingerbereich von Kindern hergestellt.
Die Schrauben haben eine Länge zwischen 5 und 20 mm. Der Gewindeaußendurchmesser beträgt dabei 1,5 mm, der Durchmesser der Innenbohrung beträgt 0,8 mm. Dadurch wird eine Fixation von Fragmenten eines gebrochenen Fingers eines Heranwachsenden möglich.

Durch die Degradation des Materials wird das Zusammenwachsen des Knochens nicht beeinträchtigt. Auch in diesem Fall erübrigt sich eine Reintervention.

### Beispiel 3:

### Ausgangsmaterial: Mg-Legierung WE 43

Vorwärts-Hohlfließpressen einer Hülse bei einer Presstemperatur von 350°C, einer Pressgeschwindigkeit von 5 mm/min ohne nachfolgende Wärmebehandlung
Das entstehende Rohr hat folgende Abmessungen:

| | |
|---|---|
| Länge (L) | = 170 mm |
| Außendurchmesser (AD) | = 2,2 mm |
| Innendurchmesser (ID) | = 0,8 mm |
| Wandstärke (WD) | = 0,7 mm |

Aus dem so prozessierten Rohr werden innenkanülierte Kirschnerdrähte gefertigt. Diese werden anschließend mit Querbohrungen (D= 0,3 mm) im Abstand von jeweils 5 mm versehen. Nach Fertigstellung des Drahtes erfolgt eine Befüllung des Innendurchmessers mit einer die Osteosynthese beschleunigenden Substanz wie z. B. nanokristallines Hydroxylapatit Pulver. Nach dem Einbringen des Kirschnerdrahtes in die Spongiosa des Oberschenkelknochens startet die Degradation der Mg-Legierung. Das mit fortschreitender Degradation des Kirschnerdrahtes ebenfalls freigesetzte Hydroxylapatit Pulver beschleunigt die Osteosynthese.

### Beispiel 4:

### Ausgangsmaterial: Mg-Legierung mit 5,0% Zn und 0,25% Ca

Vorwärts-Hohlfließpressen einer Hülse bei einer Presstemperatur von 200°C, einer Pressgeschwindigkeit von 3 mm/min ohne nachfolgende Wärmebehandlung
Das so gefertigte Rohr zeigt im Zugversuch folgende mechanische Eigenschaften:
Rₘ= 445 MPa; R_{p0,2} = 267 MPa; Aₜ= 5,0%
Das Rohr hat folgende Abmessungen:

| | |
|---|---|
| Länge (L) | = 170 mm |
| Außendurchmesser (AD) | = 2,0 mm |
| Innendurchmesser (ID) | = 0,7 mm |
| Wandstärke (WD) | = 0,65 mm |

Das Rohr weist durch die Einstellung verschiedener Umformgrade bei den innen- und außenoberflächennahen Bereichen unterschiedliche Korngrößen auf. In diesem Fall haben die außenoberflächennahen Bereiche eine mittlere Korngröße von > 15 µm. Die innenoberflächennahen Bereiche zeigen dagegen mittlere Korngrößen von < 2 µm. Dadurch entsteht ein Gradient in den mechanischen Eigenschaften. Diese wirken sich folgendermaßen aus:

Die Streckgrenze in den außenoberflächennahen Bereichen ist um ca. 50 MPa geringer als die integrale - über den gesamten Bauteilquerschitt gemessene - Streckgrenze. Dadurch wird eine nachträgliche Warmverformung der Außenoberfläche möglich. Dies findet seine Anwendung im Warmwalzen eines Außengewindes das nur durch diese Prozesskette möglich wird. Dabei wird die Hülse auf einen Innendorn geschoben und das Außengewinde im Temperaturbereich zwischen 190°C und 240°C eingewalzt. Die entstehende Spongiosaschraube kann mit einem um 30% erhöhten Drehmoment in den Knochen eingeschraubt werden als Knochenschrauben deren Gewinde spanend und ohne die vorangegangene Prozesskette aus derselben Legierung gefertigt wurden.

Weitere Ausführungsformen dieses Beispiels sind innenkanülierte Kortikalisschrauben und innenkanülierte Spongiosaschrauben für Kleinfragmente im Fingerbereich von Kindern hergestellt. Die Schrauben haben eine Länge zwischen 6 und 20 mm. Der Gewindeaußendurchmesser beträgt dabei 1,8 mm, der Durchmesser der Innenbohrung beträgt 1,2 mm. Die Gewindetiefe beträgt 0,3 mm Dadurch wird eine Fixation von Fragmenten eines gebrochenen Fingers eines Heranwachsenden möglich. Durch die Degradation des Materials wird das Zusammenwachsen des Knochens nicht beeinträchtigt. Auch in diesem Fall erübrigt sich eine Reintervention.

## Patentansprüche

1. Halbzeug für ein Implantat, umfassend oder bestehend aus einem Bereich aus einer Mg-Legierung, der durch einen Korngrößengradienten der Mg-Legierung zwischen zwei gegenüberliegen Oberflächen von ≤ 3 µm bis ≥ 8 µm bevorzugt von ≤ 2 µm bis ≥ 10 µm bezogen jeweils auf die mittlere Korngröße gekennzeichnet ist.

2. Halbzeug nach Anspruch 1, wobei das Halbzeug ein Flächengebilde oder ein Hohlprofil darstellt.

3. Halbzeug nach Anspruch 1 oder 2, wobei die Mg-Legierung bioresorbierbar ist.

4. Halbzeug nach einem der vorangehenden Ansprüche, wobei das Halbzeug in einem Extrusionsverfahren hergestellt ist.

5. Halbzeug nach einem der vorangehenden Ansprüche, wobei das Halbzeug ein Hohlprofil in Form eines Zylinders ist und die Außenfläche des Zylindermantels die Legierung mit der größeren mittleren Korngröße umfasst oder daraus besteht und die Innenfläche des Zylindermantels die Legierung mit der kleineren mittleren Korngröße umfasst oder daraus besteht.

6. Halbzeug nach einem der Ansprüche 1 bis 4, wobei das Halbzeug ein Hohlprofil in Form eines Zylinders ist und die Außenfläche des Zylindermantels die Legierung mit der kleineren mittleren Korngröße umfasst oder daraus besteht und die Innenfläche des Zylindermantels die Legierung mit der größeren mittleren Korngröße umfasst oder daraus besteht.

7. Halbzeug nach einem der vorangehenden Ansprüche, wobei das Halbzeug im Bereich der Mg-Legierung eine Zugfestigkeit von ≥ 400 bevorzugt ≥ 440 MPa und/oder eine Streckgrenze von ≥ 225 bevorzugt ≥ 250 MPa besitzt.

8. Halbzeug nach einem der vorangehenden Ansprüche, wobei das Halbzeug im Bereich der Mg-Legierung eine Bruchdehnung ≥ 4% bevorzugt ≥ 4,5% besitzt.

9. Halbzeug nach einem der vorangehenden Ansprüche, wobei die Streckgrenze des Bereiches der Mg-Legierung mit der kleineren mittleren Korngröße um ≥ 40 MPa, bevorzugt ≥ 45 MPa größer ist als die Streckgrenze des Bereiches der Mg-Legierung mit der größeren mittleren Korngröße.

10. Implantat hergestellt oder herstellbar aus einem Halbzeug nach einem der Ansprüche 1 bis 9.

11. Implantat nach Anspruch 10, wobei das Implantat im Bereich der Mg-Legierung mit der größeren mittleren Korngröße ein Gewinde oder radial verlaufende Oberflächenrillen zur Oberflächenvergrößerung aufweist.

12. Implantat nach Anspruch 10 oder 11, ausgewählt aus der Gruppe bestehend aus innenkanülierten Knochenschrauben, pico-Pin Schrauben, Knochenklammern, Marknägeln, Intramedschienen, Cerclagedraht, die Wirbelsäule versteifenden Drahtkäfigen.

13. Verwendung eines Halbzeuges nach einem der Ansprüche 1 bis 9 zur Herstellung eines Implantates.

14. Verfahren zur Herstellung eines Halbzeugs nach einem der Ansprüche 1 bis 9 umfassend die Schritte:
a) Bereitstellen einer Mg-Legierung,
b) Bereitstellen einer Extrusionsvorrichtung für die Legierung, die so ausgestaltet ist, dass beim Extrudieren zwei gegenüberliegende Flächen aus Legierungsbestandteilen entstehen, die einer unterschiedlichen Extrusionsgeschwindigkeit unterworfen waren und
c) Extrudieren der Legierung mittels der Vorrichtung.

15. Verfahren zur Herstellung eines Implantates nach einem der Ansprüche 10 bis 12 umfassend die Schritte:
a) Bereitstellen eines Halbzeuges nach einem der Ansprüche 1 bis 9 und
b) Erstellen des Implantates aus dem Halbzeug.
